# EUROPEAN PATENT SPECIFICATION

(11) **EP 0 979 652 B1**
(45) Date of publication and mention of the grant of the patent: **24.05.2006**
(21) Application number: 98945530.8
(22) Date of filing: 30.09.1998
(51) Int. Cl.: A61K 31/557, A61P 27/06

(54) **REMEDIAL COMPOSITION FOR INTRAOCULAR HYPERTENSION OR GLAUCOMA**
HEILENDE ZUSAMMENSETZUNG FÜR INTRAOKULARE HYPERTENSION ODER GLAUKOM
COMPOSITION CURATIVE DE L'HYPERTENSION INTRAOCULAIRE OU DU GLAUCOME

(30) Priority: 13.10.1997 JP 27854097
(43) Date of publication of application: 16.02.2000
(73) Proprietor: R-Tech Ueno, Ltd., Osaka-shi, Osaka 541-0043 (JP)
(72) Inventor: UENO, Ryuji, Potomac, Montgomery MD 20854 (US)
(74) Representative: Atkinson, Peter Birch
(86) International application number: PCT/JP1998/004399
(87) International publication number: WO 1999/018969

(56) References cited:
- EP-A- 0 603 800
- WO-A-97/23225
- JP-A- 3 501 025
- JP-A- 6 040 919
- TORIS C.B. ET AL: "Prostaglandins: A new class of aqueous outflow agents." OPHTHALMOLOGY CLINICS OF NORTH AMERICA, (26-09-1997) 10/3 (335-355). , XP000997423

## Description

### FIELD OF THE INVENTION

The Present invention provides a composition for treatment of ocular hypertension or glaucoma. Particularly, the invention provides a composition, which can enhance the effect of known compounds synergistically and can reduce the occurrence of unfavorable side effects.

### RELATED ART

Isopropyl unoprostone (common name), a compound which is used as a component (a) of the present invention, is (+)-isopropyl-Z-7-[(1R,2R,3R,5S)-3,5-dihydroxy-2-(3-oxodecyl)cyclopentyl] hept-5-enoate. An ocular hypotensive activity of this compound was described in EP-A-0308135 (corresponds to JP-A-02-108). Latanoprost (common name), a compound which is used as a component (b) of the present invention, is (+)-isopropyl-Z-7-{(1R, 2R, 3R, 5S)-3,5-dihydroxy-2-[(3R)-3-hydroxy-5-phenylpentyl] cyclopentyl}-5-heptenoate. An ocular hypotensive activity of this compound was described in EP-A-0364417 (corresponds to JP-A-03-501025).

However, any prior art does not teach the combined administration of the components (a) and (b) of the present invention, the effect may be increased synergistically, nor the adverse side effect may be reduced by the combination.

### SUMMARY OF THE INVENTION

An object of the present invention is to provide a composition for treatment of ocular hypertension or glaucoma, especially a composition which can provide a synergistic effect and reduce adverse side-effects.

During an extensive study about possibility to improve the effect by combining the component (a) of the present invention with a variety of compounds, the inventor surprisingly found that the effect can be synergistically enhanced by combining with the component (b) of the present invention, and that adverse side effects can be reduced by such administration. Thus, the present invention provides a composition useful for treatment of ocular hypertension or glaucoma comprising; (a) isopropyl unoprostone, and (b) Latanoprost.

Isopropyl unoprostone (common name), the compound used for component (a), is (+)-isopropyl-Z-7-[(1R,2R,3R,5S)-3,5-dihydroxy-2-(3-oxodecyl)cyclopentyl] hept-5-enoate. This compound has an ocular hypotensive activity, and different from natural PGs, does not induce transient increase of intraocular pressure. Therefore, the composition of the present invention may be applied for treatment of various diseases and conditions in which reduction of intraocular pressure is desired, for example, glaucoma, ocular hypertension and the other diseases accompanied by increased intraocular pressure. The term "treatment or treating" in this specification and claims refers to any means of control, including preventing or curing the disease, relieving or alleviating the condition, and arresting the development of the condition.

Latanoprost (common name), the compound used for component (b) in the present invention, is (+)-isopropyl-Z-7-{(1R,2R,3R,5S)-3,5-dihydroxy-2-[(3R)-3-hydroxy-5-phenylpentyl] cyclopentyl}-5-heptenoate. This compound has been known to have an ocular hypotensive activity, and also to have some adverse side effects such as transient increase of intraocular pressure, hyperemia of conjunctiva, ocular irritation, and iris pigmentation.

According to the present invention, the combined administration of the component (a) and (b) provides a synergistic effect. That is, since the combination enhances the desired effects of each of the components (a) and (b) synergistically, the dose of the each component can be reduced, and therefore, adverse side effects of the each component can be effectively reduced.

In the present composition, the ratio of (a): (b) is not limited but in general about 1:0.005-1:2, preferably, about 1:0.01-1.

The dose of each component in the present composition may vary depending on state of the patient to be treated, severity of the condition, object of the treatment, diagnosis by a doctor, and total amount of administration. Typically, the amount of the component (a) in the composition is about 0.005-2 wt%, preferably, 0.01-1 wt%. The amount of the component (b) in the composition is about 0.0001-2 wt%, preferably, 0.0005-1 wt%. The components (a) and (b) may be administered either simultaneously or individually.

The composition of the present invention may be formulated to contain both of the components (a) and (b) in a single dosage unit, or may be a package consisting of separate dosage units containing each component respectively. The composition of the present invention may further contain any conventional compound used in ophthalmologic composition such as carrier and adjuvant.

The composition of the present invention may be formulated as liquids such as solution, emulsion, and suspension, or as semisolids such as gel or ophthalmic ointment.

Examples of diluent for an aqueous solution or suspension include distilled water and physiological saline. Examples of diluent for a non-aqueous solution or suspension include vegetable oil, liquid paraffin, mineral oil, propylene glycol and p-octyldodecanol and the like. In addition, isotonic agents, such as sodium chloride, boric acid and sodium citrate to make isotonic with the lacrimal fluid, and buffering agents, such as borate buffer and phosphate buffer to maintain pH about 5.0 to 8.0 may be contained in the present composition. Further, stabilizing agents such as sodium sulfite and propylene glycol, chelating agents such as sodium edetate, thickening agents such as glycerin, carboxymethyl cellulose and carboxyvinyl polymer, and preservatives such as methyl paraben, propyl paraben may be contained in the composition. The ingredients are sterilized by means of, for example, passing through a bacterial filter or heating.

The ophthalmic ointment may contain, such as, vaseline, Zelen 50, Plastibase and Macrogol as base components and a surfactant for increasing hydrophilicity. Further, the composition may contain gelling agents such as carboxymethylcellulose, methylcellulose, carboxyvinyl polymer, as well.

In addition, the composition of the present invention may contain antibiotics such as chloramphenicol and penicillin, for preventing or treating bacterial infection.

The present invention further provides the use of (a) isopropyl unoprostone and (b) Latanoprost for manufacturing a pharmaceutical composition for treatment of ocular hypertension or glaucoma.

### BRIEF DESCRIPTION OF THE DRAWINGS

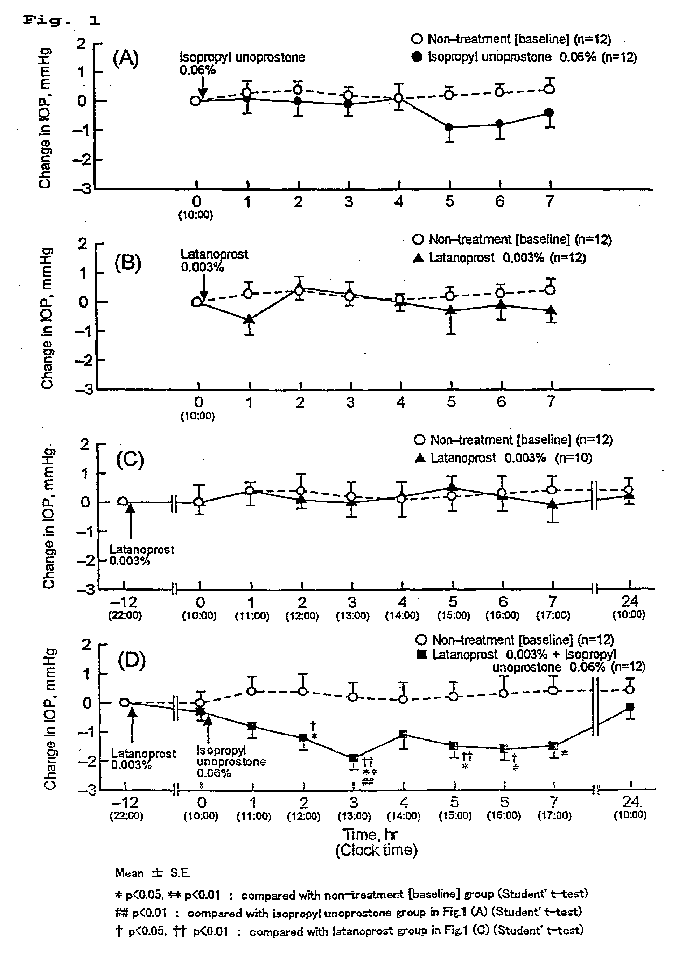

Figure 1 (A) shows a time course of intraocular pressure followed by a single instillation of 0.06 % isopropyl unoprostone eye drops.
Figure 1(B) shows a time course of intraocular pressure followed by a single instillation of 0.003 % Latanoprost eye drops at 10 o'clock.
Figure 1(C) shows a time course of intraocular pressure followed by a single instillation of 0.003% Latanoprost eye drops at 22 o'clock.
Figure 1(D) shows a time course of intraocular pressure followed by the combined instillation of 0.003% Latanoprost eye drops at 22 o'clock and 0.06% isopropyl unoprostone eye drops at the time 12 hours after that.

### THE BEST MODE OF PERFORMING THE PRESENT.

The present invention will be illustrated in more detail by the following examples.

### EXAMPLE

### Test example

### 1. Test Substance

In order to study the ocular hypotensive effect of the combined administration of isopropyl unoprostone and Latanoprost, 0.06% isopropyl unoprostone eye drops and 0.003% Latanoprost eye drops were prepared separately. Each concentration of the active ingredients is about a half of conventional clinical composition.

### 2. Animal

Male normal cynomolgus monkeys (body weight of 4.5-6.5 kg) having good health and good fore eye condition were used.

### 3. Administration schedule

Each of isopropyl unoprostone and Latanoprost eye drops were administrated topically to the respective monkeys by means of micro pipette (Gilson) in an amount of µl per eye at 10 o'clock. For clinical use, Latanoprost has been directed to administrate once a day at night, and it has been reported, based on an examination with normal volunteers, that the maximum decrease of intraocular pressure will occur about 12 hours after the administration. Therefore, in this example, the effect of Latanoprost eye drops on intra ocular pressure when administrated at 22 o'clock was also determined. For the combined administration of isopropyl unoprostone and Latanoprost, Latanoprost eye drops were administrated at 22 o'clock and then isopropyl unoprostone eye drops were administrated at 10 o'clock in the next morning, that is 12 hours after Latanoprost administration.

### 4. Measurement of Intraocular Pressure

The monkeys were anesthetized intramuscularly with 5.0-7.5 mg/kg of Ketamin (Sankyo Pharmaceuticals Co. Ltd.). A time course of the intraocular pressure was determined under topical anesthetization by dropping 0.4 % oxybuprocaine hydrochloride (Santen Pharmaceutical Co Ltd.) to eyes, by means of applanation pneumatonograph (Nippon Alcon).

### 5. Results

Changes in intraocular pressures (Δ IOP) of each administration group from the pressure determined before the administration and are shown in Fig. 1(A)-(D).

Single ocular administration of 0.06 % isopropyl unoprostone eye drops did not show any significant change in IOP when compared with a control (non-treatment) group (Fig. 1(A)).

Single ocular administration of 0.003% Latanoprost at 10 o'clock (Fig. 1(B)) and at 22 o'clock (Fig. 1(C)) did not show any significant change in IOP when compared with the control (non-treatment) group.

On the contrary, when 0.003% Latanoprost eye drops were administrated at 22 o'clock and 0.06 % isopropyl unoprostone eye drops were administrated at 10 o'clock in the next morning, that is 12 hours after Latanoprost administration (combined administration), the combination showed a significant decrease of IOP compared with any one of the groups of control (non-treatment), single administration of isopropyl unoprostone, and single administration of Latanoprost (Fig. 1 (D)).

From the above results, it was showed that the combined administration of isopropyl unoprostone and Latanoprost decreases intraocular pressure synergistically.

From the above results, it can be said that a synergistic ocular hypotensive effect will be obtained from the combination of the component (a) and (b).

### INDUSTRIAL APPLICABILITY

The present invention is useful for treatment of ocular hypertension or glaucoma.

## Claims

1. A composition for treatment of ocular hypertension or glaucoma, which comprises; (a) isopropyl unoprostone and (b) Latanoprost.

2. The composition of Claim 1, wherein said (a) isopropyl unoprostone and (b) Latanoprost are contained in a single dosage unit.

3. The composition of Claim 1, wherein said (a) isopropyl unoprostone and (b) Latanoprost are contained in separate dosage units respectively.

4. Use of (a) Isopropyl unoprostone and (b) Latanoprost for manufacturing a pharmaceutical composition for treatment of ocular hypertension or glaucoma.

5. The use of claim 4, wherein said pharmaceutical composition contains (a) isopropyl unoprostone and (b) Latanoprost in a single dosage unit.

6. The use of claim 4, wherein said pharmaceutical composition contains (a) isopropyl unoprostone and (b) Latanoprost in separate dosage units respectively.

## Patentansprüche

1. Zusammensetzung zur Behandlung von Augenhypertension oder Glaukom, die (a) Unoproston-Isopropyl und (b) Latanoprost umfasst.

2. Zusammensetzung nach Anspruch 1, wobei das (a) Unoproston-Isopropyl und das (b) Latanoprost in einer Einzeldosisierungseinheit enthalten sind.

3. Zusammensetzung nach Anspruch 1, wobei das(a) Unoproston-Isopropyl bzw. das (b) Latanoprost in getrennten Dosierungseinheiten enthalten sind.

4. Verwendung von (a) Unoproston-Isopropyl und (b) Latanoprost zum Herstellen einer pharmazeutischen Zusammensetzung zur Behandlung von Augenhypertension oder Glaukom.

5. Verwendung nach Anspruch 4, wobei die pharmazeutische Zusammensetzung (a) Unoproston-Isopropyl und (b) Latanoprost in einer Einzeldosisierungseinheit enthält.

6. Verwendung nach Anspruch 4, wobei die pharmazeutische Zusammensetzung (a) Unoproston-Isopropyl bzw. (b) Latanoprost in getrennten Dosierungseinheiten enthält.

## Revendications

1. Composition pour le traitement de l'hypertension oculaire ou du glaucome, comprenant (a) de l'isopropyle d'unoprostone et (b) du atanoprost.

2. Composition selon la revendication 1, dans laquelle lesdits (a) isopropyle d'unoprostone et (b) atanoprost sont contenus dans une seule dose unitaire.

3. Composition selon la revendication 1, dans laquelle lesdits (a) isopropyle d'unoprostone et (b) atanoprost sont respectivement contenus dans des doses unitaires distinctes.

4. Utilisation de (a) isopropyle d'unoprostone et (b) atanoprost pour la fabrication d'une composition pharmaceutique pour le traitement de l'hypertension oculaire ou du glaucome.

5. Utilisation selon la revendication 4, dans laquelle ladite composition pharmaceutique contient (a) de l'isopropyle d'unoprostone et (b) du atanoprost sous la forme d'une seule dose unitaire.

6. Utilisation selon la revendication 4, dans laquelle ladite composition pharmaceutique contient (a) de l'isopropyle d'unoprostone et (b) du atanoprost sous la forme, respectivement, de doses unitaires distinctes.
